# EUROPÄISCHE PATENTANMELDUNG

(11) **EP 4 371 580 A1**
(43) Veröffentlichungstag der Anmeldung: **22.05.2024**
(21) Anmeldenummer: 22207955.0
(22) Anmeldetag: 17.11.2022
(51) Int. Cl.: A61L 2/00, A61L 2/03, A61C 17/00

(54) **AUFSATZ ZUM REINIGEN VON IMPLANTATEN**

(71) Anmelder: Justus-Liebig-Universität Gießen, 35390 Gießen (DE)
(72) Erfinder: Harz, Torben, 35396 Gießen (DE); Jahnke, Alexander, 35633 Lahnau (DE); Rickert, Markus, 35398 Gießen (DE)
(74) Vertreter: Stumpf, Peter

(57) **Zusammenfassung**

Die Erfindung betrifft einen Aufsatz (100) zum Reinigen und Entfernen eines Biofilms von Implantaten wenigstens umfassend
• einen Anschluss (1) zum Anschließen an eine Grundeinheit (200) zur Bereitstellung von Fluid und elektrischer Energie mit einen Adapter (1a) zum Einbringen von Fluid um den Aufsatz (100) mit Fluid aus der Grundeinheit (200) zu versorgen und einen Adapter (1b) zum Einbringen elektrischer Energie aus der Grundeinheit (200) in den Aufsatz (100)
• einen fluiddurchströmbaren Stab (2) mit einer wenigstens einer ersten Öffnung (2a) in Richtung des zu reinigenden Implantats (500) und einer zweiten Öffnung (2b) zum Verbinden des Stabes (2) mit dem Adapter (1a) des Anschluss (1), wobei sich zwischen der wenigstens einen ersten Öffnung (2a) und der zweiten Öffnung (2b) sich ein Hohlraum (2c) befindet, durch welchen ein Fluid frei strömen kann, wobei der fluiddurchströmbare Stab (2) dabei mit dem Anschluss (1) so verbindbar ist, dass ein Fluid durch den Adapter (1a) über die zweite Öffnung (2b) und den Hohlraum (2c) durch die wenigstens eine erste Öffnung (2a) strömen kann
• wobei der Stab (2) eine integrierte erste elektrische Leitung (3) mit einer freiliegenden Kathode (3a) umfasst, welche dabei mit dem Minuspol des Adapters (1b) des Anschlusses (1) so verbindbar ist, dass Strom über den Aufsatz (100) zwischen der Grundeinheit (200) und einem Implantat (500) fließen kann.

Der Aufsatz (100) umfasst weiterhin eine zweite elektrische Leitung (4) mit einer Anode 4a), welche dabei mit dem Pluspol des Adapters (1b) des Anschlusses (1) so verbindbar ist, dass Strom von der ersten elektrischen Leitung über ein Implantat zur elektrische Leitung (4) fließen kann.

## Beschreibung

Die vorliegende Erfindung betrifft einen Aufsatz zum Reinigen von Implantaten.

### Beschreibung und Einleitung des allgemeinen Gebietes der Erfindung

Implantate sind im Körper eingepflanzte künstliche Materialen, die permanent oder zumindest für einen längeren Zeitraum dort verbleiben sollen. Dabei unterscheidet man häufig nach medizinischen, plastischen und funktionellen Implantaten. Die Oberflächen moderner Implantate sind mit durch verschiedene Techniken hergestellten mikrorauen Strukturen versehen, welche die Anlagerung von Osteoblasten verbessern. Ein Nachteil dieser Oberflächenstrukturen liegt in der Problematik, dass es im Falle einer mikrobielle Kontamination schwierig ist, das Implantat in situ zu dekontaminieren, ohne es aus dem Körper zu entfernen.

Diese mikrobiellen Kontaminationen führen zu Biofilmen. Biofilme sind mikrobielle Lebensgemeinschaften, die sich aus Bakterien und Pilzen zusammensetzen können. Die Mikroorganismen synthetisieren und sezernieren eine schützende Matrix, welche den Biofilm fest an einer organischen oder unbelebten Oberfläche verankert. Die in Biofilmen lebenden Mikroorganismen sind oftmals die Ursache von Fremdkörper-assoziierten Infektionen. Hierunter fallen die mikrobielle Kontamination und Besiedlung von Kathetern, medizinischen Instrumenten und gerade auch Implantaten. Besonders wegen der Affinität verschiedener Mikroorganismen, wie einigen Staphylokokken, zu den Oberflächen von Biomaterialien sind etwa die Hälfte der nosokomialen Infektionen auf chirurgische Implantate zurückzuführen. Als Ausgangspunkt der beteiligten Mikroorganismen gelten die Hautoberfläche von Krankenhauspersonal und Patienten, der Kontakt von Austrittsstellen oder Konnektoren mit Leitungswasser und weitere Quellen aus der Umgebung. Auch die Wasserleitungen von Krankenhäusern und zahnärztlichen Behandlungseinheiten sowie Dialyse-Ausrüstung und schwer zu reinigende Endoskope können betroffen sein. Abhängig von dem verwendeten Medizinprodukt und der Verweildauer kommen grampositive, gramnegative Bakterien und Pilze als Einzel- oder Multi-Spezies-Biofilm vor. Beispiele für häufig beteiligte Erreger sind:
Borrelia burgdorferi, andere humanpathogene Arten von Borrelien (B. garinii, B. afzelii, B. valaisiana, B. lusitaniae und B. spielmanii), Staphylococcus epidermidis Staphylococcus aureus, Pseudomonas aeruginosa, Escherichia coli und Candida albicans.

Aufgrund der erhöhten Antibiotika-Resistenz der Bakterien im Biofilm ist in vielen Fällen die Entfernung des jeweiligen Implantats erforderlich. Besonders gefährdet sind Systeme mit großen Oberflächen und mit Hautdurchtrittsstellen. Beispiele für häufig von Fremdkörper-assoziierten Infektionen betroffene Medizinprodukte sind: Venenkatheter, künstliche Herzklappen, Gelenkprothesen, Peritonealdialyse-Katheter, Herzschrittmacher, Endotrachealtubi, Stimmprothesen, Zerebrospinalflüssigkeit-Shunts und Zahnimplantate.

Im Zuge der Biofilmreifung kommt es zum Ablösen größerer Bakterienansammlungen. Dadurch entsteht eine Quelle für Keime, die zu chronischen und wiederkehrenden Infektionen von Patienten (Bakteriämie) und unter Umständen bis hin zur häufig tödlichen Sepsis führt. Leider ist es bisher oftmals nicht möglich den Biofilm auf einer Implantatoberfläche, bei nicht frei zugänglichen Implantaten vollständig zu entfernen, ohne, das Implantat zu explantieren.

Deshalb ist das Auftreten eines Biofilms auf einem Implantat häufig ein Grund dieses zu entfernen (Revision).

Revisionsoperationen sind leider sehr häufig. Es werden inzwischen nur in Deutschland pro Jahr allein 42 000 Revisionsoperationen alleine an Hüfte und Knie vorgenommen. Damit ist ungefähr jeder zehnte Totalendoprotheseneingriff der Revision, häufig auf Grund eines Biofilms, geschuldet.

Bei einem sog. bakteriellen Frühinfekt eines Implantates erfolgt eine Revision von infizierten Weichteilen, eine Spülung und Debridement des infizierten Gelenkes und das Auswechseln sog. mobiler Teile, wie Inlays bei Knieendoprothesen oder Hüftkopfkugeln und Pfanneninlays bei Hüftendoprothesen. Das knöchern verankerte Implantat wird möglichst belassen. Dieses gilt in vergleichbarer Weise für Osteosyntheseverfahren in der Traumatologie.

Bei einem Misserfolg dieser Behandlung, der sich in erster Linie am fortbestehenden Infekt auf Grundlage des persistierenden Biofilms misst, wird die Entfernung und ein sekundärer Wiedereinbau eines neuen Implantats erforderlich.

Deshalb ist es von großer Wichtigkeit, Implantate in situ von Biofilmen befreien zu können, ohne sie entfernen zu müssen.

### Stand der Technik

Es existieren bereits Lösungen zum Reinigen von frei zugänglichen Dentalimplantaten. Dabei wird der Biofilm bzw. die Keime ausgehend von der Implantatoberfläche getötet und/oder entfernt, indem in einem als Behandlungsflüssigkeit dienenden Fluid gelöste Ionen (Kationen und/oder Anionen) mittels elektrostatischer Kräfte durch den Biofilm hindurch zur Implantatoberfläche befördert werden um dort den Biofilm zu entfernen und so das Implantat zu reinigen.

Die keimtötende Wirkung dieses Prozesses basiert auf unterschiedlichen Effekten. Zum einen werden durch das Anlegen einer elektrischen Spannung Ionen aus dem Biofilm selbst (auch aus den Bakterien) zur Anode oder Kathode befördert. Dies kann zur Abtötung von Bakterien führen. Darüber hinaus können die Ionen, während sie den Biofilm passieren, biochemische Reaktionen eingehen, was ebenfalls zur Abtötung von Bakterien führen kann. Eine weitere Möglichkeit der Abtötung besteht darin, dass die an der Implantatoberfläche neu gebildeten Stoffverbindungen antibakterielle und/oder antivirale und/oder antifungizide Wirkung besitzen. Dies kann natürlich auch passieren, wenn die Ionen in den atomaren Zustand übergehen.

In der Schrift EP3323380A1 wird ein Behandlungssystem zur Reinigung eines mit Biofilm verunreinigten Bauteils, insbesondere zur Reinigung von bakteriell verunreinigten Oberflächen von Knochenimplantaten oder Dentalimplantaten vorgeschlagen.

Die Schrift US2016000947A1 beschreibt ein Reinigungssystem zur Entfernung eines Biofilms auf einem Implantat. Dieses System dient dabei auch der Reinigung bereits implantierter Implantate.

Die Schrift WO2016023998 beschreibt ein Reinigungssystem für ein in den Kieferknochen eines Patienten eingebrachtes Dentalimplantat.

Diese Systeme sind aber aufgrund ihres Aufbaus nicht geeignet, nicht freiliegende Endoprothesen / Schrauben / Osteosyntheseplatten vom Biofilm zu reinigen. Dies liegt u.a. daran, dass die Geometrie des Adapters nicht geeignet ist, um in tiefer liegende Gewebeschichten, z.B. bei Revision einer Hüftprothese, vorzudringen. Weiterhin lässt die geometrische Form des beschriebenen Systems es nicht zu, einen Stromfluss an die komplexen und schwer zugänglichen Strukturen von Endoprothesen, Osteosyntheseplatten etc. zu realisieren.

### Aufgabe

Aufgabe der vorliegenden Erfindung ist es, mit Biofilm kontaminierte Implantate insbesondere auch Endoprothesen und Osteosynthesen am gesamten Stütz- und Bewegungsapparat, reinigen zu können d.h. den Biofilm entfernen zu können und hierdurch einen Ausbau oder Austausch der Implantate möglichst zu vermeiden.

### Lösung der Aufgabe

Diese Aufgabe wird erfindungsgemäß gelöst durch einen Aufsatz zum Reinigen von Implantaten, der an eine Grundeinheit angeschlossen werden kann, um die Oberfläche von Implantaten wie z.B. Endoprothesen / Schrauben / Osteosyntheseplatten von Biofilm zu reinigen.

Der Aufsatz 100 zum Reinigen von Implantaten umfasst dabei einen Anschluss 1 zum Anschließen an eine Grundeinheit 200. Diese Grundeinheit 200 ist so ausgebildet, dass sie ein Fluid mit Ionen und elektrische Energie zum Betrieb des Aufsatzes 100 bereitstellen kann. Somit können beim Anlegen einer Spannung diese Ionen dann durch den Biofilm an einer Implantatoberfläche befördert werden.

Der Anschluss 1 ist dabei so ausgebildet, dass er an eine Grundeinheit 200 angeschlossen werden kann. Er umfasst dazu wenigstens einen Adapter 1a um den Aufsatz 100 mit Fluid (z.B. Wasser) aus der Grundeinheit 200 zu versorgen und wenigstens einen Adapter 1b mit einem Pluspol und einem Minuspol, um den Aufsatz 100 mit elektrischer Energie aus der Grundeinheit 200 zu versorgen. Im Sinne der klinischen Praktikabilität wird dabei ein möglichst einfaches Anschlusssystem z.B. Klicktechnik o.ä. präferiert, sodass die Adapter auch als Einmalprodukt verwendet werden können.

Weiterhin umfasst der Aufsatz 100 einen fluiddurchströmbaren Stab 2 mit wenigstens einer ersten Öffnung 2a in Richtung des zu reinigenden Implantats und einer zweiten Öffnung 2b zum Verbinden des Stabes 2 mit dem Adapter 1a des Anschluss 1. Der fluiddurchströmbaren Stab 2 kann auch mehrere Öffnungen 2a aufweisen. Größe und Anzahl der ersten Öffnungen 2a in Richtung des zu reinigenden Implantats sind abhängig von der Fließgeschwindigkeit und der Fluidaustrittsmenge der Grundeinheit. Typtische Flussraten liegen im Bereich von ca. 100 ml je min Die Anzahl der Öffnungen liegt vorzugsweise bei 1 bis 10 und ihre Größe zwischen 0,1 mm und 2 mm.

Die Größe des Stabs 2 richtet sich nach dem Operationsfeld und liegt üblicherweise im Bereich 5 bis 20 cm. Die Größe des Aufsatzes 100 kann je nach Einsatz und zugrundeliegender Operationstechnik variieren. Als Material wird ein üblicher OP geeigneter Kunststoff bevorzugt. Zwischen der wenigstens einen ersten Öffnung 2a und der zweiten Öffnung 2b befindet sich ein Hohlraum 2c. Durch diesen Hohlraum 2c kann ein Fluid frei strömen.

Der fluiddurchströmbare Stab 2 ist dabei mit dem Anschluss 1 so verbindbar, dass ein Fluid durch den Adapter 1a über die zweite Öffnung 2b und den Hohlraum 2c durch wenigsten eine erste Öffnung 2a strömen kann.

Dabei umfasst der Stab 2 eine in den Stab integrierte erste elektrische Leitung 3 mit einer freiliegenden Kathode 3a. Diese erste elektrische Leitung 3 ist dabei mit dem Minuspol des Adapters 1b des Anschlusses 1 so verbindbar, dass Strom über den Aufsatz 100 zwischen der Grundeinheit und einem Implantat 500 fließen kann. Weiterhin umfasst der Aufsatz 100 eine zweite elektrische Leitung 4 mit der Anode 4a. Die zweite elektrische Leitung 4 ist mit dem Pluspol des Adapters 1b des Anschlusses 1 so verbindbar, sodass Strom von der ersten elektrischen Leitung über ein Implantat und das Fluid zur zweiten elektrische Leitung 4 fließen kann.

Die elektrischen Leitungen 3, 4 sind dabei beispielsweise in Form von Kupfer- und/oder Aluminiumdrähten, -kabeln oder -litzen ausgeführt.

Die Kathode 3a ist so ausgebildet, dass sie in direkten Kontakt zu dem Implantat 500 gebracht kann. Die Anode 4 weist dabei einen Abstand von mindestens 1mm zur freiliegenden Kathode 3a auf. Dabei ist die Anode 4 so ausgebildet, dass sie stets wenigstens 1mm Abstand zum Implantat 500 aufweist, sodass der Stromfluss im eingeschalteten Zustand der Kathode 3a über das Implantat 500 zur Anode 4 stattfinden kann, und eine möglichst geringe Fluidsäule notwendig ist und so der Stromfluss über das Fluid optimal ist.

### Weitere Ausführungsformen:

In einer weiteren besonderen Ausführungsform umfasst der Aufsatz 100 zum Reinigen von Implantaten noch eine Bürste aus einzelnen Lamellen, diese ist dabei am Stab 3 angeordnet. Dabei ist in wenigstens einer Lamelle eine erste elektrische Leitung 3 mit einer freiliegenden Kathode 3a angeordnet. Als Material für die Bürste werden Silikon, Latex o.ä. biotolerante Materialen eingesetzt.

Vorzugsweise umfasst der Aufsatz 100 zum Reinigen von Implantaten eine Haube 8 zum Aufsetzen auf ein Implantat. Diese Haube 8 ist dabei um den Stab 2 herum abgeordnet, sodass sich beim Aufsetzen des Aufsatzes 100 zum Reinigen von Implantaten auf ein Implantat eine Haubenkammer 8b ausbildet. Als Material für die Haube werden Silikon, Latex o.ä. biotolerante Materialen verwendet. Die Größe ist implantatabhängig und kann nach dem "Kondomprinzip" unterschiedlich ausfallen. Die Haube 8 ist ausgebildet, sodass sie das Implantat möglichst dicht umschließen kann. Die Haubenkammer 8b ist der Raum zwischen Implantat 500 und Haube 8 in welchem das Fluid zum Reinigen des Implantats eingebracht werden kann. Diese Begrenzung durch die Haube 8 senkt den Bedarf an Fluid und schützt das Gewebe, welches das Implantat umgibt, vor Irritationen durch das Fluid.

Dabei umfasst die Haube 8 einen Dichtungsring 8a. Als Material für die Dichtung werden Silikon, Latex o.ä. biotolerante Materialen verwendet. Dieser ist an der dem Stab 2 abgewandten Seite der Haube 8 angeordnet. Er dient zur Abdichtung der Haubenkammer 8b, wenn der Aufsatz 100 zum Reinigen von Implantaten auf einem Implantat aufsitzt.

Die zweite elektrische Leitung 4 ist im Dichtungsring 8a der Haube 8 angeordnet. sodass Strom von der ersten elektrischen Leitung 3 mit einer freiliegenden Kathode 3a über das Fluid zum Implantat 500 und die Haubenkammer zur elektrischen Leitung 4 mit der Anode 4a im Dichtungsring 8a fließen kann.

Bei dem hier beschriebenen Aufsatz handelt es sich um ein System, das eingesetzt werden kann, um den Biofilm auf einem Implantat zu entfernen. Dieses Implantat kann so einer Revisionsoperation zur Entfernung des Bewuchs mit Biofilm unterzogen werden, ohne das gesamte Implantat explantieren zu müssen. Das ist für die Patienten natürlich deutlich schonender und verkürzt auch deren Liegezeit im Krankenhaus.

### Verfahrensbeschreibung:

Zum Betrieb des Aufsatzes 100 wird dieser in einem Schritt A zunächst über die Adapter 1a und 1b mit der Grundeinheit 200 verbunden. Anschließend wird der Anschuss in einem Schritt B, so zum Implantat 500 positioniert, dass Strom von der ersten elektrischen Leitung 3 mit einer freiliegenden Kathode 3a über das Fluid zum Implantat 500 zur elektrischen Leitung 4 und der Anode 4a im Dichtungsring 8a fließen kann.

Anschließend wird in einem Schritt C ein als Behandlungsflüssigkeit dienendes Fluid mit einer ausreichenden Konzentration beweglicher Ladungsträger über den Adapter 1a durch den Stab 2 und dessen zweite Öffnung 2b in den Zwischenraum zwischen dem Stab 2 der freiliegenden Kathode 3a und dem Implantat 500 eingeleitet.

Grundbestandteil des Fluids ist ein Metallsalz in wässriger Lösung. Besonders bevorzugt wird eine Lösung mit dem Metallsalz Natriumformiat verwendet. Das Metallsalz liefert die Ionen für den Stromtransport. Durch die gezielte Wahl einer ausreichend hohen elektrischen Leitfähigkeit soll sichergestellt werden, dass der Stromfluss durch das Fluid und damit durch die behandlungsbedürftigen Teile und Komponenten, nicht aber durch das Körpergewebe des Patienten erfolgt, so dass eine Gefährdung des Patienten durch einen ungewollten Stromfluss durch Weichgewebe, Knochen, Blut und/oder andere Körpermaterialien minimiert werden kann. Die elektrische Leitfähigkeit der Behandlungsflüssigkeit sollte dabei möglichst ein Vielfaches der elektrischen Leitfähigkeit von Blut, Knochen, Weichgewebe, Fettgewebe oder anderen Körpermaterialien aufweisen. Daher sollte die elektrische Leitfähigkeit der Behandlungsflüssigkeit einen Wert von mindestens 30 mS/cm aufweisen.

Das Einleiten des Fluids geschieht dabei vorzugsweise kontinuierlich. Die Durchflussmenge kann dabei variabel an der Grundeinheit eingestellt werden, um einen kontinuierlichen Fluss zu gewährleisten. Die Flussrate hängt dabei von der Größe des zu reinigenden Implantats und dem entsprechender Adapter ab. Anschließend wird zwischen dem Pluspol und dem Minuspol am Adapter 1a eine Spannungsdifferenz angelegt. Die Spannung liegt dabei vorzugsweise zwischen 10 V und 20 V.

Die Stromstärke liegt zwischen 0,1 und 1A. Dann fließen Ladungsträger vom Minuspol des Adapters 1a über die erste Leitung 3, die Kathode 3a und das Fluid zum Implantat 500. Dabei werden im Fluid 500 Ionen erzeugt. Diese Ionen reagieren an der Implantatoberfläche chemisch oder elektrochemisch mit dem Biofilm. Durch diese Reaktionen werden neue Stoffverbindungen geschaffen und/oder die Ionen selbst und/oder Teile dieser Ionen in den atomaren Zustand überführt. Dies führt zu einer Entfernung des Biofilms. Je nach Fluid im Implantatmaterial reagieren die Ionen des Fluids auch mit dem Oberflächenmaterial (z.B. Bildung einer Oxidschicht oder Materialabtrag), was den Reinigungseffekt verstärkt oder eine Wiederbesiedlung erschwert. Anschließend fließt Strom vom Implantat über die zweite elektrische Leitung zum Pluspol des Adapters 1a. Dies erfolgt solange bis der Biofilm hinreichend entfernt wurde.

Die "Bürsten"- und die "Pfannenausführung" können bewegt werden, um einen zusätzlichen Reinigungseffekt zu realisieren, die anderen Adapter sind ausgebildet nicht bewegt zu werden.

Die Fluidrückführung wird durch eine externe Absaugung, wie sie auch für andere Operationen (z.B. Implantationen) verwendet wird, realisiert.

Das Fluid mit den Resten des Biofilms wird während und nach dem Reinigungsvorgang über standardmäßige medizinische Absaugsysteme abgesaugt.

Mit diesem Aufsatz 100 ist es möglich ein vollständiges Reinigungssystem 300, mit dem Aufsatz 100 und einer Grundeinheit 200 zur Versorgung des Aufsatzes 100 mit einen Fluid und elektrischer Energie zu konstruieren.

### Ausführungsbeispiele

Für die konkrete Ausgestaltung des Aufsatzes 100 gibt es verschiedene Ausführungsformen. Diese sind nur als beispielhafte Ausführungsvarianten zu verstehen und stellen keine abschließende Auflistung aller Ausführungsformen dar.

### 1 ."Hüfte"-Ausführung

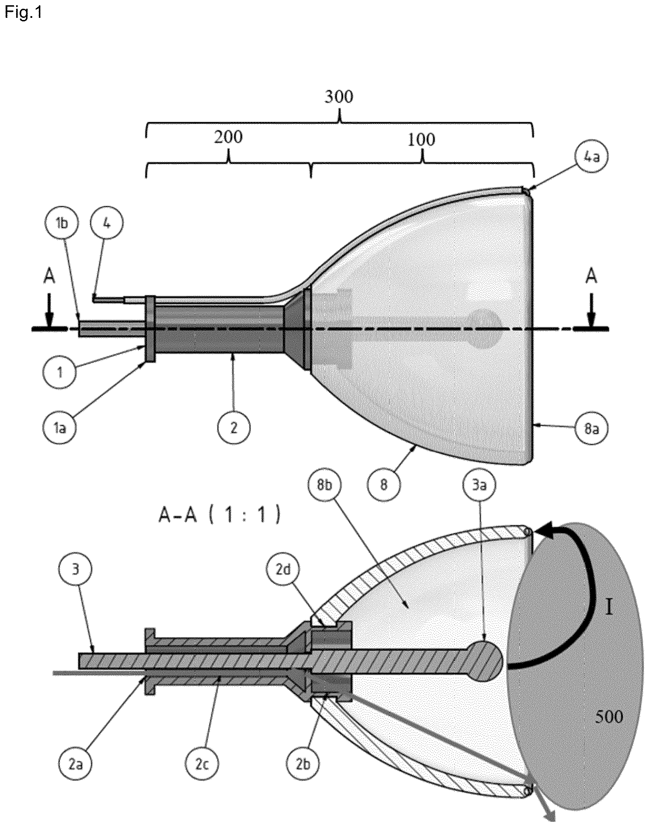

Schematisch wird dieses Prinzip durch Fig.1 dargestellt. Das Grundprinzip dieses Aufsatzes besteht aus einem Anschluss 1 mit dem Stab 2 der als Griff fungiert und an die Grundeinheit 200 angeschlossen wird. Innerhalb dieses Stabes befindet sich ein Hohlraum 2c durch den das als Reinigungsflüssigkeit dienende Fluid laufen kann. Ebenfalls befindet sich in diesem Stab eine als Kathoden-Kontaktdraht ausgeführte erste elektrische Leitung 3 um einen elektrischen Kontakt zwischen der Grundeinheit 200 und dem metallischen Implantat zu gewährleisten. Am unteren Ende der ersten elektrischen Leitung 3 befindet sich eine Kathoden-Metallspitze 3a mit Kugelkopf. Am Ende des Stabes 2 befinden sich Öffnungen 2b in Form von Austrittslöchern, durch welche das Fluid in die flexible Haube 8 einfließen kann. Diese Haube 8 kann in verschiedenen Größen ausgebildet sein und über den Knochen (z.B. proximales oder distales Femur, proximale Tibia etc.) einschließlich der einliegenden Implantate übergestülpt werden. Ferner ist ein flexibler Dichtungsring 8a vorgesehen, um das zu reinigende Implantat vom angrenzenden Gewebe abzugrenzen. In diesem Dichtungsring befindet sich die als Anoden-Kontaktdraht ausgebildete zweite elektrische Leitung 4, um eine möglichst geringe Fluidsäule zu realisieren. Dies führt zu einer besseren Stromzufuhr zwischen dem Implantat 500 als Kathode und der zweiten elektrischen Leitung 4 und der Anode 4a innerhalb der Haubenkammer 8b.

Diese Ausführungsform ist besonders für Implantate, die eine "Überstülpung" des Knochens zulassen (z.B. proximales Femur) geeignet.

### 2."Bürsten"-Ausführung:

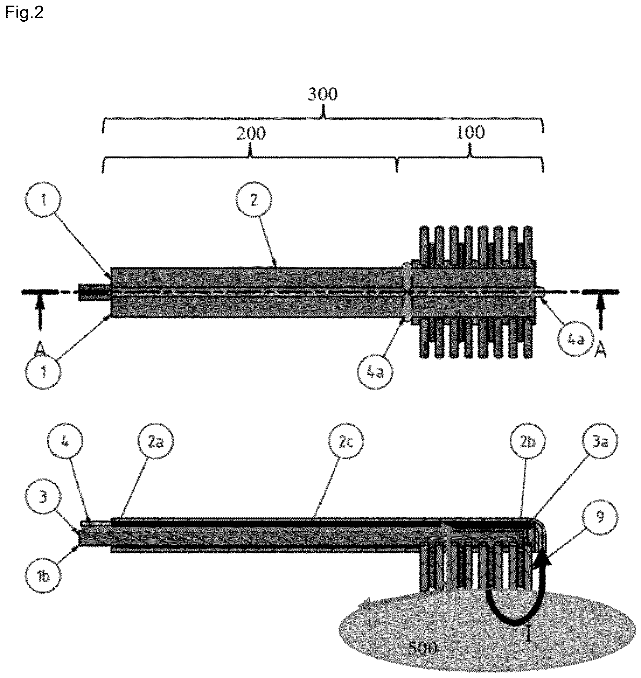

Schematisch wird dieses Prinzip durch Fig.2 dargestellt. Der Anschluss 1 mit dem Stab 2 entspricht dem Stab der "Hüfte"-Ausführung. Der Anschluss 1 mit dem Stab 2, der Hohlraum 2c und die als Kathoden-Kontaktdraht ausgeführte erste elektrische Leitung 3 funktionieren wie in der "Hüfte"-Ausführung. Am unteren Ende des Aufsatzes ist in der "Bürsten"-Ausführung: eine Bürste mit Lamellen 9 ausgebildet. In wenigstens einem Teil dieser Lamellen liegt die erste elektrische Leitung 3 mit Drahtkathoden 3a, welche flexibel ist und somit zu allen Richtungen einen Stromfluss zu einem Implantat herstellen kann. Über Austrittsöffnungen 2b am Ende des Stabes 2, kann das Fluid zum Implantat gelangen.

Diese Ausführungsform kann insbesondere für Knieendoprothesen, aber auch für sämtliche Implantate, die leicht zugänglich sind, verwendet werden.

### 3. "Pfannen"-Ausführung:

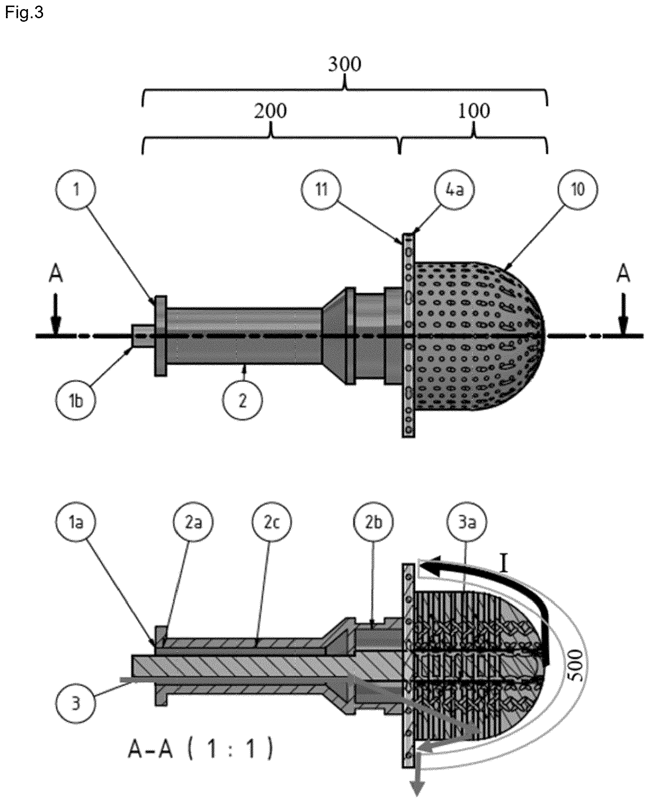

Schematisch wird dieses Prinzip durch Fig.3 dargestellt. Das Grundprinzip dieses Aufsatzes basiert ebenfalls auf der Ausführungsform "Hüft"-Aufsatz. Es besteht aus einem Anschluss 1 mit einem Stab 2. Dabei ist der Anschluss 1 mit einer Grundeinheit 200 verbindbar. Innerhalb dieses Stab befindet sich ein Hohlraum 2c durch den das Fluid laufen kann. Ebenfalls befindet sich in diesem Stab eine als Kathoden-Kontaktdraht ausgeführte erste elektrische Leitung 3, um einen elektrischen Kontakt zwischen der Grundeinheit 200 und dem metallischen Implantat 500 zu gewährleisten. Am unteren Ende der ersten elektrischen Leitung 3 befindet sich eine Kathode 3a, welche in Form einer Metallspitze mit Kugelkopf ausgebildet ist. Über diese Kathode 3a wird der Elektronenfluss zwischen dem Aufsatz 100 und dem Implantat 500 realisiert. Am Ende des Stabes 2 befinden sich Austrittsöffnungen 2b mit einem Schwamm 10 in welchen das Fluid austreten kann. Der Schwann besteht vorzugsweise aus aufgeschäumten Kunststoff - z.B. Polyurethan. Die Aufgabe ist es Fluid gleichmäßig innerhalb des Pfannenadapters zu verteilen und einen kontinuierlichen Stromfluss zu realisieren.

Optional umfasst diese Ausführungsvariante eine Haubenarretierungsnut 2d an dem Anschluss 1 entgegengesetzten Ende des Stabes 2 und eine mit dieser Haubenarretierungsnut 2d verbundenen Haube 8.

Mit dieser Haube kann das Implantat 500 zusätzlich abgedichtet werden. Dies entspricht der flexiblen Haube beim "Hüft"-Aufsatz.

Weiterhin umfasst die Ausführungsvariante eine Abdeckung 11, welche zwischen dem Stab 2 und dem Schwamm 10 angeordnet ist. Die Abdeckung 11 ist in verschiedenen Größen ausführbar und dient dazu das Implantat 500 (z.B. eine Hüftpfanne) an den umliegenden Knochen anzupressen, um eine besonders stabile Verbindung zwischen dem Aufsatz 100 mit dem Implantat 500 herstellen. Unterhalb der Abdeckung 11 befindet sich die als Mesh-Anodendraht ausgeführte zweite elektrische Leitung 4.

### 4. "Platten"-Ausführung:

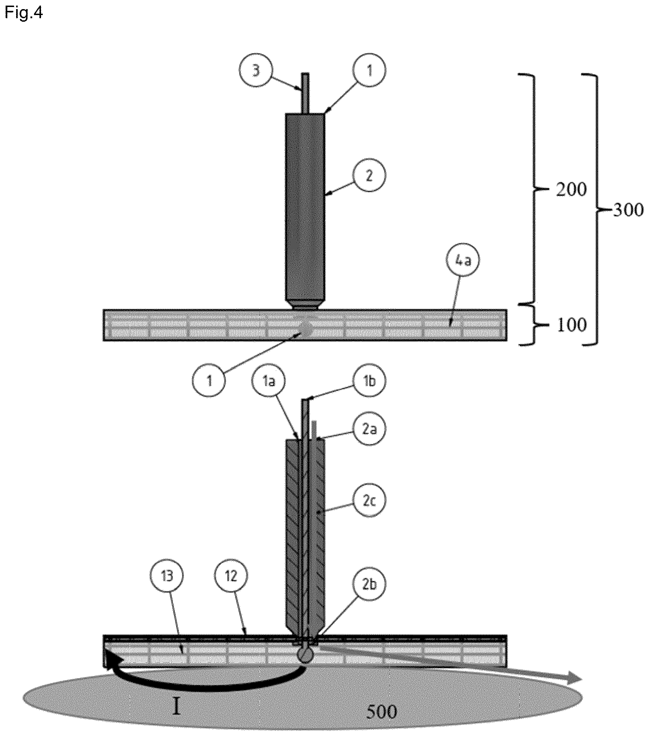

Schematisch wird dieses Prinzip durch Fig.4 dargestellt. Die "Platten"-Ausführung weist einen Anschluss 1 mit einem Stab 2 zum Anschluss an eine bestehende Grundeinheit 200 auf. Innerhalb dieses Stabs befindet sich ein fluiddurchströmbarer Hohlraum 2c. Diese Ausführungsform des Aufsatzes 100 umfasst eine halbrunde Schiene 12 mit innen anliegender als Mesh-Anodendraht ausgeführter zweiter elektrischer Leitung 4 über die gesamte Länge. Diese Schiene 12 ist mit dem Stab 2 über dessen Öffnung 2b des Stabes 2 fluiddurchströmbar verbunden. So kann sich bei der Reinigung ein Fluid aus dem Stab 2 über die Öffnung 2b innerhalb der Schiene 12 und der Schienenkammer 12a verteilen, um ein plattenflörmiges Implantat (z.B. Plattenosteosynthese) zu benetzen.

Die Schienenkammer 12a ist der Raum zwischen Implantat 500 und Schiene 12, in welchem das Fluid zum Reinigen des Implantats eingebracht werden kann. Sie erfüllt die gleiche Aufgabe wie die Haubenkammer 8b in Ausführungsvarianten des Aufsatzes 100 mit einer Haube 8.

Die Schiene 12 kann dabei auch noch direkt vor der Reinigung gekürzt und so an die Länge der jeweiligen Platte angepasst werden.

Diese Ausführungsform umfasst eine im Stab 2 angeordnete, als Kathoden-Kontaktdraht ausgeführte erste elektrische Leitung 3. Diese dient dazu einen elektrischen Kontakt zwischen der Grundeinheit 200 und dem Implantat 500 zu gewährleisten.

Diese Ausführungsform umfasst eine innerhalb der Schiene 12 angeordnete, als Mesh-Anodendraht ausgeführte zweite elektrische Leitung 4.

Diese Ausführungsform ist speziell für flache Implantate (z.B. Plattenimplantate, Knochenplatten, Fixationsplatten) optimiert.

### Abbildungslegenden und Bezugszeichenliste

**Fig. 1** zeigt eine "Hüfte"-Ausführung des Aufsatzes 100 mit einer Haube 8.
**Fig. 2** zeigt eine "Bürsten"-Ausführung des Aufsatzes 100 mit Lamellen 9, welche eine Bürste bilden.
**Fig. 3** zeigt eine "Pfannen"-Ausführung des Aufsatzes 100 mit einem Schwamm 10 und einer Abdeckung 11.
**Fig. 4** zeigt eine "Platten"-Ausführung Aufsatzes 100 mit einer Schiene 12 und einer innerhalb der Schiene 12 liegende Schienenkammer 12a.

In allen Abbildungen Fig.1 bis Fig.4 zeigt I die Stromrichtung an. Der Pfeil zeigt dabei die Flussrichtung negativ geladener Ladungsträger, wenn Strom fließt.

### Bezugszeichenliste

1 Anschluss
1a erster Adapter zur Zuführung von Fluid
1b zweiter Adapter zur Zuführung von elektrischer Energie
2 Stab
2a, 2b Öffnung im Stab
2c Hohlraum
2d Haubenarretierungsnut
3 erste elektrische Leitung
3a Kathode
4 zweite elektrische Leitung
4a Anode
8 Haube
8a Dichtungsring
8b Haubenkammer
9 Lamellen
10 Schwamm
11 Abdeckung
12 Schiene
12a Schienenkammer
100 Aufsatz zum Reinigen von Implantaten
200 Grundeinheit
300 Reinigungssystem
500 Implantat
I Stromfluss
A Figurenachse

## Patentansprüche

1. Aufsatz (100) zum Reinigen von Implantaten und Entfernen eines Biofilms wenigstens umfassend
• einen Anschluss (1) zum Anschließen an eine Grundeinheit (200) zur Bereitstellung von Fluid und elektrischer Energie
o wobei der Anschluss (1) einen ersten Adapter (1a) zum Einbringen von Fluid um den Aufsatz (100) mit Fluid aus der Grundeinheit (200) zu versorgen und einen zweiten Adapter (1b) zum Einbringen elektrischer Energie aus der Grundeinheit (200) in den Aufsatz (100) aufweist
• einen fluiddurchströmbaren Stab (2) mit einer ersten Öffnung (2a) zum Verbinden des Stabes (2) mit dem Adapter (1a) des Anschluss (1) und einer zweiten Öffnung (2a) in Richtung des zu reinigenden Implantats (500),
∘ wobei sich zwischen der wenigstens einen ersten Öffnung (2a) und der zweiten Öffnung (2b) ein Hohlraum (2c) befindet, durch welchen ein Fluid frei strömen kann, wobei der fluiddurchströmbare Stab (2) dabei mit dem Anschluss (1) so verbindbar ist, dass ein Fluid durch den Adapter (1a) über die zweite Öffnung (2b) und den Hohlraum (2c) durch die wenigstens eine erste Öffnung (2a) strömen kann und
∘ wobei der Stab (2) eine integrierte erste elektrische Leitung (3) mit einer freiliegenden Kathode (3a) umfasst, welche dabei mit dem Minuspol des Adapters (1b) des Anschlusses (1) so verbindbar ist, dass Strom über den Aufsatz (100) zwischen der Grundeinheit (200) und einem Implantat (500) fließen kann
**dadurch gekennzeichnet, dass**
der Aufsatz (100) weiterhin eine zweite elektrische Leitung (4) mit einer Anode (4a) umfasst, welche dabei mit dem Pluspol des Adapters (1b) des Anschlusses (1) so verbindbar ist, dass Strom von der ersten elektrischen Leitung über ein Implantat (500) zur elektrische Leitung (4) fließen kann.

2. Aufsatz (100) gemäß Anspruch 1, **dadurch gekennzeichnet, dass** der Aufsatz (100) noch eine Bürste aus einzelnen Lamellen (9), welche am Stab (3) angeordnet sind, umfasst, wobei in wenigstens einer Lamelle die erste elektrische Leitung (3) mit einer freiliegenden Kathode (3a) angeordnet ist.

3. Aufsatz (100) gemäß Anspruch 1 oder 2 **dadurch gekennzeichnet, dass** der Aufsatz (100) eine Haube (8) zum Aufsetzen auf ein Implantat umfasst, die um den Stab (2) herum so angeordnet ist, sodass beim Aufsetzen des Aufsatzes (100) auf ein Implantat eine Haubenkammer (8b) entsteht.

4. Aufsatz (100) gemäß Anspruch 3 **dadurch gekennzeichnet, dass** die Haube (8) einen Dichtungsring (8a) umfasst, welcher an der dem Stab (2) abgewandten Seite der Haube (8) angeordnet ist.

5. Aufsatz (100) gemäß Anspruch 3 **dadurch gekennzeichnet, dass** die zweite elektrische Leitung (4) im Dichtungsring (8a) der Haube (8) angeordnet ist, sodass Strom von der ersten elektrischen Leitung (3) über ein Implantat und die Haubenkammer (8b) zur zweiten elektrische Leitung (4) fließen kann.

6. Aufsatz (100) gemäß Anspruch 1 **dadurch gekennzeichnet, dass** der Aufsatz (100) eine halbrunde Schiene (12) mit innen anliegender als Mesh-Anodendraht ausgeführter zweiter elektrischer Leitung (4) über die gesamte Länge umfasst, wobei die Schiene (12) mit dem Stab (2) über dessen Öffnung (2b) fluiddurchströmbar verbunden ist.

7. Reinigungssystem (300), **dadurch gekennzeichnet, dass** dieses wenigstens einen Aufsatz (100) gemäß einem der vorhergehenden Ansprüche sowie eine Grundeinheit (200) zur Versorgung des Aufsatzes (100) mit einen Fluid und elektrischer Energie umfasst.
